# EUROPÄISCHE PATENTSCHRIFT

(11) **EP 1 429 768 B1**
(45) Veröffentlichungstag und Bekanntmachung des Hinweises auf die Patenterteilung: **16.11.2016**
(21) Anmeldenummer: 02777013.0
(22) Anmeldetag: 06.09.2002
(51) Int. Cl.: A61K 31/439, A61K 9/00, A61K 31/137

(54) **NEUE ARZNEIMITTEL ZUR INHALATION**
NOVEL MEDICAMENTS FOR INHALATION
AGENTS PHARMACEUTIQUES D'INHALATION

(30) Priorität: 14.09.2001 DE 10145438; 04.03.2002 DE 10209243
(43) Veröffentlichungstag der Anmeldung: 23.06.2004
(73) Patentinhaber: Boehringer Ingelheim Pharma GmbH & Co. KG, 55216 Ingelheim am Rhein (DE)
(72) Erfinder: LINZ, Günter, 88441 Mittelbiberach (DE); SOYKA, Rainer, 88400 Biberach (DE)
(86) Internationale Anmeldenummer: PCT/EP2002/009974
(87) Internationale Veröffentlichungsnummer: WO 2003/024452

(56) Entgegenhaltungen:
- WO-A-00/69468
- WO-A-02/38154
- REES P J: "BRONCHODILATORS IN THE THERAPY OF CHRONIC OBSTRUCTIVE PULMONARY DISEASE" EUROPEAN RESPIRATORY MONOGRAPH, EUROPEAN RESPIRATORY SOCIETY JOURNALS LTD., SHEFFIELD,, GB, Bd. 7, Nr. 7, 1998, Seiten 135-149, XP000937584 ISSN: 1025-448X
- BARNES P J: "Chronic obstructive pulmonary disease: new opportunities for drug development" TRENDS IN PHARMACOLOGICAL SCIENCES, ELSEVIER TRENDS JOURNAL, CAMBRIDGE, GB, Bd. 19, Nr. 10, Oktober 1998 (1998-10), Seiten 415-423, XP004156947 ISSN: 0165-6147

## Beschreibung

Die vorliegende Erfindung betrifft neue Arzneimittel zur Inhalation auf der Basis von Tiotropiumsalzen und schwerlöslichen Salzen des Salmeterols, Verfahren zu deren Herstellung sowie deren Verwendung bei der Therapie von Atemwegserkrankungen.

### Hintergrund der Erfindung

Die Verbindung Tiotropiumbromid, ein Salz des Tiotropiums, ist aus der Europäischen Patentanmeldung EP 418 716 A1 bekannt und weist die folgende chemische Struktur auf:

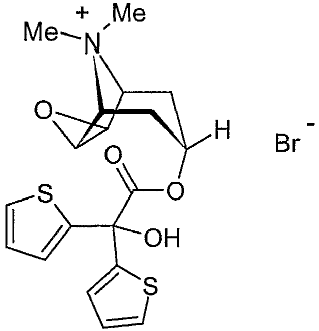

Diese Verbindung kann auch auch durch den chemischen Namen (1α,2β,4β,5α,7β)-7-[(hydroxydi-2-thienylacetyl)oxy]-9,9-dimethyl-3-oxa-9-azoniatricyclo[3.3.1.0^{2,4}] nonane-bromid bezeichnet werden und besitzt wertvolle pharmakologische Eigenschaften. Die Bezeichnung Tiotropium ist im Rahmen der vorliegenden Erfindung als Bezugnahme auf das freie Kation zu verstehen.

Sie, wie auch andere Salze des Tiotropiums, stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumsalzen erfolgt vorzugsweise auf inhalativem Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten. Die inhalative Applikation kann ferner mittels geeigneter Lösungen des Tiotropiumsalzes erfolgen.

WO 00/69468 offenbart Arzneimittelzusammensetzungen zur Verwendung bei der Therapie von Atemwegserkrankungen.

### Detaillierte Beschreibung der Erfindung

Überraschenderweise wurde gefunden, daß ein unerwartet vorteilhafter therapeutischer Effekt, insbesondere ein synergistischer Effekt bei der Behandlung von entzündlichen oder obstruktiven Atemwegserkrankungen beobachtet wird, wenn ein oder mehrere Tiotropiumsalze (**1**) in Kombination mit ein oder mehreren schwerlöslichen Salmeterolsalzen (**2**) zur Anwendung gelangen, wobei die Salmeterolsalze **2** in Wasser eine Löslichkeit von 0,1 mg/ml oder weniger, bevorzugt 0,05 mg/ml oder weniger, besonders bevorzugt 0,035 mg/ml oder weniger aufweisen. Diese im Rahmen der vorliegenden Erfindung zur Anwendung gelangenden Salmeterolsalze **2** sind insbesondere durch eine hohe lokale Verträglichkeit gekennzeichnet.

Hierdurch lassen sich beispielsweise unerwünschte Nebenwirkungen, die häufig bei der Applikation von β-Mimetika, wie Salmeterol, am Menschen beobachtet werden deutlich verringern. Als zentrale Nebenwirkungen von β-Mimetika seien beispielsweise allgemeine Unruhe, Erregung, Schlaflosigkeit, Angst, Fingerzittern, Schweißausbrüche und Kopfschmerzen genannt.

Dementsprechend betrifft die vorliegende Anmeldung Arzneimittelkombinationen gekennzeichnet durch den Gehalt an einem oder mehreren, bevorzugt einem Tiotropiumsalz (**1**) in Kombination mit einem oder mehreren, bevorzugt einem schwerlöslichen Salmeterolsalz (**2**), wobei das Salmeterolsalz **2** in Wasser eine Löslichkeit von 0,1 mg/ml oder weniger, bevorzugt 0,05 mg/ml oder weniger, besonders bevorzugt 0,035 mg/ml oder weniger aufweist.

Insbesondere betrifft die vorliegende Erfindung Arzneimittel gekennzeichnet durch den Gehalt an einem oder mehreren Tiotropiumsalzen (**1**) in Kombination mit einem oder mehreren schwerlöslichen Salmeterolsalzen (**2**), wobei die Salmeterolsalze **2** ausgewählt sind aus den Säureadditionssalzen des Salmeterols **2**' mit einer der nachstehend genannten Säuren: 2-Hydroxy-1-naphthoesäure, 3,5-Dichlorsalicylsäure, Furan-2-carbonsäure, 2,5-Dihydroxy-terephthalsäure, Zimtsäure, Triphenylessigsäure, 4-Phenylzimtsäure, Biphenyl-2-carbonsäure, 4-Trifluormethylzimtsäure, 9-Fluoronylidenessigsäure, 3-(2-Naphthyl)acrylsäure, 3-(1-Naphthyl)acrylsäure, 1-Naphthoesäure, 2,6-Dichlorbenzoesäure, 3,4-Dichlorbenzoesäure, 3,5-Dichlorbenzoesäure, 4-Brombenzoesäure, 4-Trifluormethylbenzoesäure, 4-lsopropylbenzoesäure, 4-tert.-Butylbenzoesäure, 3-(3-Indolyl)acrylsäure, 2,4-Dichlorzimtsäure, 2,6-Dichlorzimtsäure, 2,5-Dimethoxyzimtsäure, 2-Trifluormethylzimtsäure, 3-Trifluormethylzimtsäure, 3-Chlorzimtsäure, 3,4-Dichlorzimtsäure, 4-Bromzimtsäure, 4-Chlorzimtsäure, 4-Methoxyzimtsäure, 4-Fluorzimtsäure, 4-lsopropylzimtsäure, 4-tert.-Butylzimtsäure, 2,6-Difluorzimtsäure, 2,4-Difluorzimtsäure, 3,4-Difluorzimtsäure, 2,4,5-Trifluorzimtsäure, 3,4,5-Trifluorzimtsäure, 3-Methoxysalicylsäure, 4-Methoxysalicylsäure, 5-Methoxysalicylsäure, 4-Methylsalicylsäure, 5-Aminosalicylsäure, 3-Chlorsalicylsäure, 5-Sulfosalicylsäure, 5-Acetylsalicylsäure, 3,5-Diiodsalicylsäure, Isochinolin-1-carbonsäure, 9-Fluorencarbonsäure, 9-Fluorenon-1-carbonsäure, 3,5-Diisopropyl-salicylsäure oder Diflunisal; und in Wasser eine Löslichkeit von 0,1 mg/ml oder weniger aufweisen.

Die Bezeichnung Tiotropium ist im Rahmen der vorliegenden Erfindung als Bezugnahme auf das freie Kation (**1'**) zu verstehen. Eine Bezugnahme auf Salmeterol ist im Rahmen der vorliegenden Erfindung als Bezugnahme auf die freie Base (**2'**) zu verstehen. Die Herstellung von Salmeterol wurde erstmals in der britischen Patentschrift GB 2140800 beschrieben, auf die hiermit inhaltlich Bezug genommen wird.

Die erfindungsgemäßen Wirkstoffkombinationen sind überraschenderweise ferner sowohl durch einen raschen Wirkungseintritt, als auch durch eine langandauernde Wirkdauer gekennzeichnet. Dies ist von hoher Bedeutung für das Wohlbefinden des Patienten, da er einerseits nach Applikation der Kombination eine rasche Verbesserung seines Zustands verspürt und andererseits aufgrund der langen Wirkdauer eine einmal pro Tag erfolgende Applikation ausreichend ist.

Die vorstehend genannten Effekte werden sowohl bei gleichzeitiger Applikation innerhalb einer einzigen Wirkstoffformulierung als auch bei sukzessiver Applikation der beiden Wirkstoff in getrennten Formulierungen beobachtet. Erfindungsgemäß bevorzugt ist die gleichzeitige Applikation der beiden Wirkstoffbestandteile in einer einzigen Formulierung.

Die vorstehend genannten Effekte werden sowohl bei gleichzeitiger Applikation innerhalb einer einzigen Wirkstoffformulierung als auch bei sukzessiver Applikation der beiden Wirkstoff in getrennten Formulierungen beobachtet. Erfindungsgemäß bevorzugt ist die gleichzeitige Applikation der beiden Wirkstoffbestandteile in einer einzigen Formulierung.

Ein Aspekt der vorliegenden Erfindung betrifft ein Arzneimittel, welches ein oder mehrere Tiotropiumsalze (**1**) und ein oder mehrere Salmeterolsalze (**2**), gegebenfalls in Form ihrer Solvate oder Hydrate enthält. Hierbei können die Wirkstoffe entweder gemeinsam in einer einzigen Darreichungsform oder in zwei getrennten Darreichungsformen enthalten sein. Erfindungsgemäß bevorzugt sind Arzneimittel, die die Wirkstoffe **1** und **2** in einer einzigen Darreichungsform enthalten.

Ein weiterer Aspekt der vorliegenden Erfindung betrifft ein Arzneimittel, welches neben therapeutisch wirksamen Mengen von **1** und **2** einen pharmazeutisch verträglichen Hilfsstoff enthält. Ein Aspekt der vorliegenden Erfindung betrifft ein Arzneimittel, welches neben therapeutisch wirksamen Mengen von **1** und **2** keinen pharmazeutisch verträglichen Hilfsstoff enthält.

Die vorliegende Erfindung betrifft ferner die Verwendung von **1** und **2** zur Herstellung eines therapeutisch wirksame Mengen von **1** und **2** enthaltenden Arzneimittels zur Behandlung von entzündlichen oder obstruktiven Atemwegserkrankungen, insbesondere von Asthma oder COPD durch simultane oder sukzessive Applikation.

Die vorliegende Erfindung zielt ferner auf die simultane oder sukzessive Verwendung therapeutisch wirksamer Dosen der Kombination vorstehender Arzneimittel **1** und **2** zur Behandlung von entzündlichen oder obstruktiven Atemwegserkrankungen, insbesondere von Asthma oder COPD.

Unter den im Rahmen der vorliegenden Erfindung einsetzbaren Tiotropiumsalzen **1** sind die Verbindungen zu verstehen, die neben Tiotropium als Gegenion (Anion) Chlorid, Bromid, Iodid, Methansulfonat, para-Toluolsulfonat oder Methylsulfat enthalten. Im Rahmen der vorliegenden Erfindung sind von allen Tiotropiumsalzen das Methansulfonat, Chlorid, Bromid oder lodid bevorzugt, wobei dem Methansulfonat oder dem Bromid besondere Bedeutung zukommt. Von erfindungsgemäß herausragender Bedeutung ist das Tiotropiumbromid. Die Tiotropiumsalze **1** können gegebenenfalls in Form ihrer Solvate und Hydrate zum Einsatz gelangen. Besonders bevorzugt werden dabei die Hydrate verwendet. Von allen Hydraten der erfindungsgemäß einsetzbaren Tiotropiumsalze **1** wird im Rahmen der vorliegenden Erfindung besonders bevorzugt das kristalline Tiotropiumbromidmonohydrat verwendet, welches in der WO 02/30928 beschrieben ist. Auf diese Internationale Patentanmeldung wird an dieser Stelle vollinhaltlich Bezug genommen.

Unter schwerlöslichen Salzen des Salmeterols **2** werden erfindungsgemäß pharmazeutisch verträgliche Salze des Salmeterols verstanden, die in Wasser eine Löslichkeit 0,1 mg/ml oder weniger, bevorzugt 0,05 mg/ml oder weniger, besonders bevorzugt 0,035 mg/ml oder weniger aufweisen.

Salmeterol weist ein Chiralitätszentrum auf. Die vorliegende Erfindung umfaßt die Salze **2** in racemischer oder enantiomerenreiner Form. Hierbei kommt sowohl dem (R)- als auch dem (S)-Enantiomer eine besondere Bedeutung zu. Ferner können im Rahmen der vorliegenden Erfindung die Salze **2** in Form der nicht racemischen Gemische der beiden Enantiomere eingesetzt werden.

Die Herstellung der Salmeterolsalze **2** ausgehend von der freien Base des Salmeterols erfolgt gemäß oder in Analogie zu im Stand der Technik für die Bildung von Säureadditionssalzen bekannten Verfahren. Sie beinhaltet die Umsetzung der freien Base Salmeterol **2'** mit den entsprechenden Carbonsäuren in geeigneten Lösemitteln, vorzugsweise in organischen Lösemitteln. Hierzu wird die gewünschte Säure vorzugsweise zunächst in einem organischen Lösemittel, besonders bevorzugt einem Lösemittel ausgewählt aus der Gruppe bestehend aus Ethylacetat, Methanol, Ethanol, iso-Propanol und Diethylether oder Mischungen davon aufgenommen. Gegebenenfalls können die vorstehend genannten Lösemittel auch in Mischungen mit tert.-Butylmethylether oder Cyclohexan zum Einsatz gelangen. Gegebenenfalls werden die in einem der vorstehend genannten Lösemittel aufgenommenen Säuren unter Erwärmen, vorzugsweise unter Erwärmen bis zur Siedetemperatur des Lösemittels gelöst. Zu dieser Lösung wird Salmeterol **2',** gegebenenfalls gelöst in einem der vorstehend genannten Lösemittel zugegeben. Aus der erhaltenen Lösung werden die Salze **2** gegebenenfalls unter Abkühlen kristallisiert und isoliert.

Erfindungsgemäß bevorzugt sind die schwerlöslichen Salmeterolsalze **2** 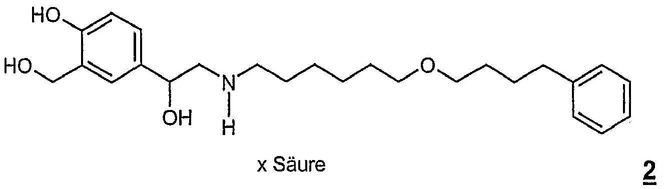 ausgewählt aus der Gruppe:
4-Hydroxy-α¹-[[[6-(4-phenylbutoxy)-hexyl]-amino]-methyl]-1,3-benzoldimethanol-biphenyl-4-carboxylat-salz;
4-Hydroxy-α¹-[[[6-(4-phenylbutoxy)-hexyl]-amino]-methyl]-1,3-benzoldimethanol-4-chlorsalicylat-salz;
4-Hydroxy-α¹-[[[6-(4-phenylbutoxy)-hexyl]-amino]-methyl]-1,3-benzoldimethanol-2,4-dihydroxybenzoat-salz;
4-Hydroxy-α¹-[[[6-(4-phenylbutoxy)-hexyl]-amino]-methyl]-1,3-benzoldimethanol-2-hydroxy-3-naphtoat-salz;
4-Hydroxy-α¹-[[[6-(4-phenylbutoxy)-hexyl]-amino]-methyl]-1,3-benzoldimethanol-1-hydroxy-2-naphtoat-salz;
4-Hydroxy-α¹-[[[6-(4-phenylbutoxy)-hexyl]-amino]-methyl]-1,3-benzoldimethanol-salicylat-salz;

Ferner bevorzugt werden erfindungsgemäß als schwerlösliche Salmeterolsalze **2** jene Salze in die erfindungsgemäßen Arzneimittelkombinationen eingesetzt, die durch Umsetzung der freien Base des Salmeterols **2'** mit einer oder mehrerer, bevorzugt einer der nachfolgend genannten Säuren erhalten werden:

| **Säure** | **Lösemittel (zur Salzbildung)** | **Schmelzpunkt 2 in °C (gemäß DSC)** |
|---|---|---|
| 2-Hydroxy-1-naphthoesäure | Essigsäureethylester | 122 |
| 3,5-Dichlorsalicylsäure | Diethylether | 108 |
| Furan-2-carbonsäure | Diethylether | 80 |
| 0,5 x 2,5-Dihydroxy-terephthalsäure | Essigsäureethylester | 102 |
| Zimtsäure | Essigsäureethylester | 89 |
| Triphenylessigsäure | Diethylether | 116 |
| 4-Phenylzimtsäure | Essigsäureethylester | 109 |
| Biphenyl-2-carbonsäure | Essigsäureethylester | 127 |
| 4-Trifluormethylzimtsäure | Essigsäureethylester | 125 |
| 9-Fluorenylidenessigsäure | Essigsäureethylester | 88 |
| 3-(2-Naphthyl)acrylsäure | Essigsäureethylester | 97 |
| 3-(1-Naphthyl)acrylsäure | Essigsäureethylester | 77 |
| 1-Naphthoesäure | Essigsäureethylester | 120 |
| 2,6-Dichlorbenzoesäure | Essigsäureethylester | 103 |
| 3,4-Dichlorbenzoesäure | Essigsäureethylester | 115 |
| 3,5-Dichlorbenzoesäure | Essigsäureethylester | 110 |
| 4-Brombenzoesäure | Essigsäureethylester | 115 |
| 4-Trifluormethylbenzoesäure | Essigsäureethylester | 125 |
| 4-lsopropylbenzoesäure | Essigsäureethylester | 85-90 |
| 4-tert.-Butylbenzoesäure | Essigsäureethylester | 95 |
| 3-(3-Indolyl)acrylsäure | Essigsäureethylester | 113 |
| 2,4-Dichlorzimtsäure | Essigsäureethylester | 138 |
| 2,6-Dichlorzimtsäure | Essigsäureethylester | 82 |
| 2,5-Dimethoxy-zimtsäure | Essigsäureethylester | 88 |
| 2-Trifluormethylzimtsäure | Essigsäureethylester | 94 |
| 3-Trifluormethylzimtsäure | Essigsäureethylester | 92 |
| 3-Chlorzimtsäure | Essigsäureethylester | 90 |
| 3,4-Dichlorzimtsäure | Essigsäureethylester | 116 |
| 4-Bromzimtsäure | Essigsäureethylester | 127 |
| 4-Chlorzimtsäure | Essigsäureethylester | 123 |
| 4-Methoxyzimtsäure | Essigsäureethylester | 98 |
| 4-Fluorzimtsäure | Essigsäureethylester | 113 |
| 4-Isopropylzimtsäure | Essigsäureethylester | 82 |
| 4-tert.-Butylzimtsäure | Essigsäureethylester | 93 |
| 2,6-Difluorzimtsäure | Essigsäureethylester | 77 |
| 2,4-Difluorzimtsäure | Essigsäureethylester | 121 |
| 3,4-Difiluorzimtsäure | Essigsäureethylester | 102 |
| 2,4,5-Trifluorzimtsäure | Essigsäureethylester | 120 |
| 3,4,5-Trifluorzimtsäure | Essigsäureethylester | 107 |
| 3-Methoxysalicylsäure | Essigsäureethylester | 118 |
| 4-Methoxysalicylsäure | Essigsäureethylester | 113 |
| 5-Methoxysalicylsäure | Essigsäureethylester | 114 |
| 4-Methylsalicylsäure | Essigsäureethylester | 116 |
| 5-Aminosalicylsäure | Essigsäureethylester/Isopropanol | 146 |
| 3-Chlorsalicylsäure | Essigsäureethylester | 108 |
| 5-Sulfosalicylsäure | Essigsäureethylester/ Isopropanol | 129 |
| 5-Acetylsalicylsäure | Essigsäureethylester | 80 |
| 3,5-Diiodsalicylsäure | Essigsäureethylester | 133 |
| Isochinolin-1-carbonsäure | Essigsäureethylester | 105 |
| 9-Fluorencarbonsäure | Essigsäureethylester | 90 |
| 9-Fluorenon-1-carbonsäure | Essigsäureethylester | 136 |
| 3,5-Diisopropyl-salicylsäure | Essigsäureethylester | 115 |
| Diflunisal | Essigsäureethylester | 104 |

Erfindungsgemäß von besonderer Bedeutung sind im Rahmen der erfindungsgemäßen Arzneimittelkombinationen die schwerlöslichen SalmeterolSalze **2** ausgewählt aus der Gruppe bestehend aus:
4-Hydroxy-α¹-[[[6-(44-phenylbutoxy)-hexyl]-amino]-methyl]-1,3-benzoldimethanol-4-phenylcinnamat-Salz **2.1:**

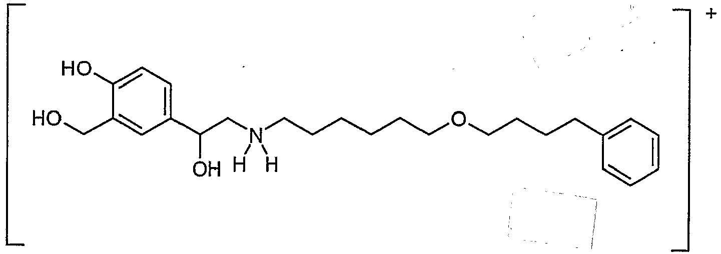

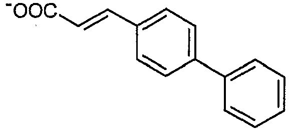

1.35 g (6 mmol) 4-Phenylzimtsäure wird in 75 mL Essigsäureethylester unter Erwärmen zum Rückfluß gelöst. Zu dieser Lösung gibt man eine warme Lösung von 2.5 g (6 mmol) Salmeterol in 25 mL Essigsäureethylester. Man läßt die Lösung abkühlen und rührt 16 h bei Raumtemperatur. Die Suspension wird filtriert, der Niederschlag mit Essigsäureethylester und tert.-Butylmethylether gewaschen und im Vakuum bei 25-30°C getrocknet. Man erhält 3.47 g der Titelverbindung als farblosen Feststoff. Schmelzpunkt: 109°C;

In analagoer Art und Weise wurden die nachfolgenden Verbindungen hergestellt:
4-Hydroxy-α¹-[[[6-(4-phenylbutoxy)-hexyl]-amino]-methyl]-1,3-benzoldimethanol-4-trifluoromethyl-cinnamat-Salz **2.2;**
   Schmelzpunkt: 125°C;
4-Hydroxy-α¹-[[[6-(4-phenylbutoxy)-hexyl]-amino]-methyl]-1,3-benzoldimethanol-3,4-dichlor-cinnamat-Salz **2.3;**
   Schmelzpunkt: 116°C;
4-Hydroxy-α¹-[[[6-(4-phenylbutoxy)-hexyl]-amino]-methyl]-1,3-benzoldimethanol-2,4-dichlor-cinnamat-Salz **2.4;**
   Schmelzpunkt: 183°C;
4-Hydroxy-α¹-[[[6-(4-phenylbutoxy)-hexyl]-amino]-methyl]-1,3-benzoldimethanol-cinnamat-Salz **2.5;**
   Schmelzpunkt: 89°C;
4-Hydroxy-a¹-[[[6-(4-phenylbutoxy)-hexyl]-amino]-methyl]-1,3-benzoldimethanol-3-(2-naphthyl)acrylat-Salz **2.6;**
   Schmelzpunkt: 97°C;
4-Hydroxy-α¹-[[[6-(4-phenylbutoxy)-hexyl]-amino]-methyl]-1,3-benzoldimethanol-3-(1-naphthyl)acrylat-Salz **2.7;**
   Schmelzpunkt: 77°C;
4-Hydroxy-α¹-[[[6-(4-phenylbutoxy)-hexyl]-amino]-methyl]-1,3-benzoldimethanol-2,6-dichlor-cinnamat-Salz **2.8;**
   Schmelzpunkt: 82°C;
4-Hydroxy-α¹-[[[6-(4-phenylbutoxy)-hexyl]-amino]-methyl]-1,3-benzoldimethanol-2,5-dimethoxy-cinnamat-Salz **2.9;**
   Schmelzpunkt: 88°C;
4-Hydroxy-α¹-[[[6-(4-phenylbutoxy)-hexyl]-amino]-methyl]-1,3-benzoldimethanol-2-trifluoromethyl-cinnamat-Salz **2.10;**
   Schmelzpunkt: 94°C;
4-Hydroxy-α¹-[[[6-(4-phenylbutoxy)-hexyl]-amino]-methyl]-1,3-benzoldimethanol-3-trifluoromethyl-cinnamat-Salz **2.11**;
   Schmelzpunkt: 92°C;
4-Hydroxy-α¹-[[[6-(4-phenylbutoxy)-hexyl]-amino]-methyl]-1,3-benzoldimethanol-3-chlor-cinnamat-Salz **2.12;**
   Schmelzpunkt: 90°C;
4-Hydroxy-α¹-[[[6-(4-phenylbutoxy)-hexyl]-amino]-methyl]-1,3-benzoldimethanol-4-brom-cinnamat-Salz **2.13**;
   Schmelzpunkt: 127°C;
4-Hydroxy-α¹-[[[6-(4-phenylbutoxy)-hexyl]-amino]-methyl]-1,3-benzoldimethanol-4-chlor-cinnamat-Salz **2.14;**
   Schmelzpunkt: 123°C;
   7
4-Hydroxy-α¹-[[[6-(4-phenylbutoxy)-hexyl]-amino]-methyl]-1,3-benzoldimethanol-4-methoxy-cinnamat-Salz **2.15**;
   Schmelzpunkt: 98°C;
4-Hydroxy-α¹-[[[6-(4-phenylbutoxy)-hexyl]-amino]-methyl]-1,3-benzoldimethanol-4-fluor-cinnamat-Salz **2.16;**
   Schmelzpunkt: 113°C;
4-Hydroxy-α¹-[[[6-(4-phenylbutoxy)-hexyl]-amino]-methyl]-1,3-benzoldimethanol-4-isopropyl-cinnamat-Salz **2.17;**
   Schmelzpunkt: 82°C;
4-Hydroxy-α¹-[[[6-(4-phenylbutoxy)-hexyl]-amino]-methyl]-1,3-benzoldimethanol-4-tert-butyl-cinnamat-Salz **2.18;**
   Schmelzpunkt: 93°C;
4-Hydroxy-α¹-[[[6-(4-phenylbutoxy)-hexyl]-amino]-methyl]-1,3-benzoldimethanol-2,4-difluor-cinnamat-Salz **2.19;**
   Schmelzpunkt: 121°C;
4-Hydroxy-α¹-[[[6-(4-phenylbutoxy)-hexyl]-amino]-methyl]-1,3-benzoldimethanol-3,4-difluor-cinnamat-Salz **2.20;**
   Schmelzpunkt: 102°C;
4-Hydroxy-α¹-[[[6-(4-phenylbutoxy)-hexyl]-amino]-methyl]-1,3-benzoldimethanol-2,4,5-trifluor-cinnamat-Salz **2.21;**
   Schmelzpunkt: 120°C;
4-Hydroxy-α¹-[[[6-(4-phenylbutoxy)-hexyl]-amino]-methyl]-1,3-benzoldimethanol-3,4,5-trifluor-cinnamat-Salz **2.22;**
   Schmelzpunkt: 107°C;

Von erfindungsgemäß gleichrangiger Bedeutung sind im Rahmen der erfindungsgemäßen Arzneimittelkombinationen die schwerlöslichen SalmeterolSalze **2** ausgewählt aus der Gruppe bestehend aus:
4-Hydroxy-α¹-[[[6-(4-phenylbutoxy)-hexyl]-amino]-methyl]-1,3-benzoldimethanol-5-(2,4-difluorphenyl)salicylat-Salz **2.23**:

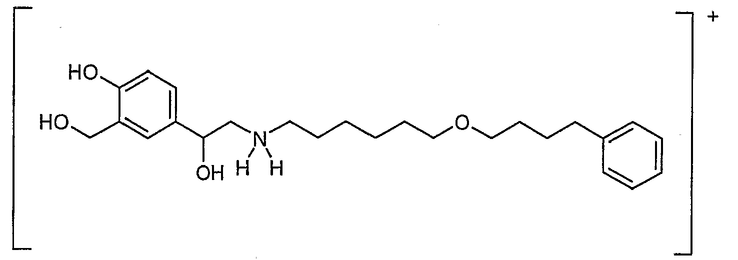

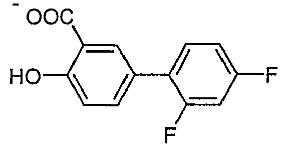

30 g Salmeterol wird unter Erwärmen zum Rückfluß in 300 mL Essigsäureethylester gelöst. Zu dieser Lösung gibt man 18.3 g 5-(2,4-difluorphenyl)salicylsäure (Diflunisal). Man läßt die Lösung auf Raumtemperatur abkühlen. Die Suspension wird abfiltriert, der Niederschlag mit Essigsäureethylester gewaschen und im Vakuum bei 35°C getrocknet. Man erhält 46 g des Titelsalzes als farblosen Feststoff Schmelzpunkt: 104°C

In analagoer Art und Weise wurden die nachfolgenden Verbindungen hergestellt:
4-Hydroxy-α¹-[[[6-(4-phenylbutoxy)-hexyl]-amino]-methyl]-1,3-benzoldimethanol-3,5-dlisopropyl-salicylat-Salz **2.24**;
   Schmelzpunkt: 115°C;
4-Hydroxy-α¹-[[[6-(4-phenylbutoxy)-hexyl]-amino]-methyl]-1,3-benzoldimethanol-4-chlor-salicylat-Salz **2.25**;
   Schmelzpunkt: 123°C;
4-Hydroxy-α¹-[[[6-(4-phenylbutoxy)-hexyl]-amino]-methyl]-1,3-benzoldimethanol-3,5-dichlor-salicylat-Salz **2.26**;
   Schmelzpunkt: 108°C;
4-Hydroxy-α¹-[[[6-(4-phenylbutoxy)-hexyl]-amino]-methyl]-1,3-benzoldimethanol-2,5-dihydroxyterephthalat-Salz **2.27;** (Base : Säure = 1:2).
   Schmelzpunkt: 102°C;
4-Hydroxy-α¹-[[[6-(4-phenylbutoxy)-hexyl]-amino]-methyl]-1,3-benzoldimethanol-3-methoxysalicylat-Salz **2.28**;
   Schmelzpunkt: 118°C;
4-Hydroxy-α¹-[[[6-(4-phenylbutoxy)-hexyl]-amino]-methyl]-1,3-benzoldimethanol-4-methoxysalicylat-Salz **2.29**;
   Schmelzpunkt: 113°C;
4-Hydroxy-α¹-[[[6-(4-phenylbutoxy)-hexyl]-amino]-methyl]-1,3-benzoldimethanol-5-methoxysalicylat-Salz **2.30**;
   Schmelzpunkt: 114°C;
4-Hydroxy-α¹-[[[6-(4-phenylbutoxy)-hexyl]-amino]-methyl]-1,3-benzoldimethanol-4-methylsalicylat-Salz **2.31**;
   Schmelzpunkt: 116°C;
4-Hydroxy-α¹-[[[6-(4-phenylbutoxy)-hexyl]-amino]-methyl]-1,3-benzoldimethanol-5-aminosalicylat-Salz **2.32**;
   Schmelzpunkt: 146°C;
4-Hydroxy-α¹-[[[6-(4-phenylbutoxy)-hexyl]-amino]-methyl]-1,3-benzoldimethanol-3-chlorsalicylat-Salz **2.33;**
   Schmelzpunkt: 108°C;
4-Hydroxy-α¹-[[[6-(4-phenylbutoxy)-hexyl]-amino]-methyl]-1,3-benzoldimethanol-5-sulfo-salicylat-Salz **2.34;**
   Schmelzpunkt: 129°C;
4-Hydroxy-α¹-[[[6-(4-phenylbutoxy)-hexyl]-amino]-methyl]-1,3-benzoldimethanol-5-acetylsalicylat-Salz **2.35**;
   Schmelzpunkt: 80°C;
4-Hydroxy-α¹-[[[6-(4-phenylbutoxy)-hexyl]-amino]-methyl]-1,3-benzoldimethanol-3,5-diiodsalicylat-Salz **2.36**;
   Schmelzpunkt: 133°C;

In den vorstehend genannten erfindungsgemäßen Salzen liegen die Base Salmeterol und die jeweils zum Einsatz gelangende Säure sofern nichts anderes angegeben ist, im molaren Verhaltnis Salmeterol:Säure 1:1 vor.

Die Identität der vorstehend genannten Verbindungen wurde mittels 1H-NMR-Spektroskopie und ESI-Massenspektrometrie bestätigt.

In den erfindungsgemäßen Wirkstoffkombinationen aus **1** und **2** können die Bestandteile **1** und **2** in Form ihrer Enantiomere, Gemische der Enantiomere oder in Form der Racemate enthalten sein.

Die Verhältnisse, in denen die beiden Wirkstoffe **1** und **2** in die erfindungsgemäßen Wirkstoffkombinationen eingesetzt werden können, sind variabel. Die Wirkstoffe **1** und **2** können gegebenfalls in Form ihrer Solvate oder Hydrate vorliegen. Je nach Wahl der Salze **1** bzw. **2** variieren die im Rahmen der vorliegenden Erfindung einsetzbaren Gewichtsverhältnisse aufgrund des unterschiedlichen Molekulargewichts der verschiedenen Salzformen. Den nachfolgend angegebenen Gewichtsverhältnissen wurden daher das Tiotropiumkation **1'** sowie die freie Base des Salmeterols **2'** zugrunde gelegt. Die erfindungsgemäßen Wirkstoffkombinationen können **1'** und **2'** in Gewichtsverhältnissen enthalten, die in einem Bereich von 1:300 bis 30:1, bevorzugt von 1:230 bis 20:1, besonders bevorzugt von 1:150 bis 10:1, ferner bevorzugt von 1:50 bis 5:1, besonders bevorzugt von 1:35 bis 2:1. Erfindungsgemäß von besonderem Interesse sind Arzneimittel, enthaltend die Kombination aus **1'** und **2**' in einem Gewichtsverhältnis im Bereich von 1:25 bis 1:1. bevorzugt in einem Bereich von 1:10 bis 1:2, besonders bevorzugt in einem Bereich von 1:5 bis 1:2,5.

Beispielsweise und ohne den Umfang der Erfindung darauf zu beschränken, können bevorzugte erfindungsgemäße Kombinationen aus **1** und **2** Tiotropium **1'** und Salmeterol **2'** in den folgenden Gewichtsverhältnissen enthalten: 1:40; 1:20; 1:11,1; 1:10; 1:5,6; 1:5; 1:2,8; 1:2,5; 1:1,4; 1:1,25; 1,44:1, 1,6:1.

Die Anwendung der erfindungsgemäßen Arzneimittel enthaltend die Kombinationen aus **1** und **2** erfolgt üblicherweise so, daß Tiotropium **1'** und Salmeterol **2'** gemeinsam in Dosierungen von 0,01 bis 10000µg, bevorzugt von 0,1 bis 2000µg, besonders bevorzugt von 1 bis 1000µg, ferner bevorzugt von 5 bis 500µg, erfindungsgemäß bevorzugt von 10 bis 200µg, bevorzugt von 20 bis 100µg, höchst bevorzugt von 30 bis 70µg pro Einmalgabe.

Beispielsweise enthalten erfindungsgemäße Kombinationen aus **1** und **2** eine solche Menge an Tiotropium **1'** und Salmeterol **2'**, daß die Gesamtdosierung pro Einmalgabe 30µg, 35µg, 45µg, 55µg, 60µg, 65µg, 90µg, 105µg, 110µg, 140µg oder ähnliches beträgt. Bei diesen Dosierungsbereichen sind die Wirkstoffe **1'** und **2'** in den vorhergehend beschriebenen Gewichtsverhältnissen enthalten.

Beispielsweise und ohne den Umfang der Erfindung darauf zu beschränken, können die erfindungsgemäßen Kombinationen aus **1** und **2** eine solche Menge an Tiotropium **1'** und Salmeterol **2'** enthalten, daß pro Einmalgabe 5µg **1'** und 25µg **2',** 5µg **1'** und50µg **2',** 5µg **1'** und 100µg **2',** 5µg **1'** und 200µg **2',** 10ug **1'** und 25µg **2',** 10µg **1'** und 50µg **2',** 10µg **1'** und 100µg **2',** 10µg **1'** und 200µg **2',** 18µg **1'** und 25µg **2',** 18µg **1'** und 50µg **2',** 18µg 1.: und 100µg **2',** 18µg **1'** und 200µg **2',** 20µg **1'** und 25µg **2',** 20µg **1'** und 50µg **2',** 20µg **1'** und 100µg **2',** 20µg **1'** und 200ug **2',** 36µg **1'** und 25µg **2',** 36µg **1'** und 50µg **2',** 36µg **1'** und 100µg **2',** 36µg **1'** und 200µg **2'**, 40µg **1'** und 25µg **2',** 40µg **1'** und 50µg **2',** 40µg **1'** und 100µg **2'** oder 40µg **1'** und 200µg **2'** appliziert werden.

Wird als erfindungsgemäß bevorzugte Kombination aus **1** und **2** diejenige Wirkstoffkombination verwendet, in der **1** Tiotropiumbromid bedeutet und in der **2** für eines der besonders bevorzugten Salze **2,** das Salmeterol-4-phenylcinnamat (2.1) steht, entsprechen die vorstehend beispielhaft genannten pro Einmalgabe applizierten Wirkstoffmengen von **1'** und **2'** den nachfolgenden pro Einmalgabe applizierten Mengen an **1** und **2** 6µg **1** und 38,49µg **2,** 6µg **1** und 76,98µg **2,** 6µg **1** und 153,96µg **2,** 6µg **1** und 307,92µg **2,** 12µg **1** und 38,49µg **2,** 12µg **1** und 76,98µg **2**, 12µg **1** und 153,96µg **2**, 12pg **1** und 307,92µg **2,** 21,7µg **1** und 38,49µg **2,** 21,7µg **1** und 76,98µg **2**, 21,7µg **1** und 153,96µg **2**, 21,7µg **1** und 307,92µg **2,** 24,1 µg **1** und 38,49µg **2,** 24,1 µg **1** und 76,98µg **2,** 24,1 µg **1** und 153,96µg **2,** 24,1 µg **1** und 307,92µg **2,** 43,3µg **1** und 38,49µg **2,** 43,3µg **1** und 76,98µg **2**, 43,3µg **1** und 53,96µg **2,** 43,3µg **1** und 307,92µg **2,** 48,1 µg **1** und 38,49µg **2,** 48,1 µg **1** und 76,98µg **2,** 48,1 µg **1** und 153,96µg **2** oder 48,1 µg **1** und 307,92µg **2.**

Wird in der erfindungsgemäß bevorzugten Kombination aus **1** und **2,** in der **2** für eines der erfindungsgemäß besonders bevorzugten Salze **2,** das Salmeterol-4-phenylcinnamat-salz **(2.1)** steht, als Komponente **1** beispielsweise das Tiotropiumbromidmonohydrat eingesetzt, entsprechen die vorstehend beispielhaft genannten pro Einmalgabe applizierten Wirkstoffmengen von **1'** und **2'** den nachfolgenden pro Einmalgabe applizierten Mengen an **1** und **2**: 6,2µg **1** und 38,49µg **2,** 6,2µg **1** und 76,98µg **2,** 6,2µg **1** und 153,96µg **2,** 6,2µg **1** und 307,92µg **2**, 12,5µg **1** und 38,49µg **2,** 12,5µg **1** und 76,98µg **2,** 12,5µg **1** und 153,96µg **2,** 12,5µg **1** und 307,92µg **2,** 22,5µg **1** und 38,49µg **2,** 22,5µg **1** und 76,98µg **2,** 22,5µg **1** und 153,96µg **2,** 22,5µg **1** und 307,92µg **2,** 25µg **1** und 38,49µg **2,** 25µg **1** und 76,98µg **2,** 25µg **1** und 153,96µg **2**, 25µg **1** und 307,92µg **2,** 45µg **1** und 38,49µg **2,** 45µg **1** und 76,98µg **2,** 45µg **1** und 153,96µg **2,** 45µg **1** und 307,92µg **2,** 50µg **1** und 38,49µg **2,** 50µg **1** und 76,98µg **2,** 50µg **1** und 153,96µg **2** oder 50µg **1** und 307,92µg **2**.

Die Applikation der erfindungsgemäßen Wirkstoffkombinationen aus **1** und **2** erfoigt bevorzugt auf inhalativem Wege. Hierzu müssen die Bestandteile **1** und **2** in inhalierbaren Darreichungsformen bereitgestellt werden.

Als inhalierbare Darreichungsformen kommen insbesondere Inhalationspulver in Betracht. Erfindungsgemäße Inhalationspulver enthaltend die Wirkstoffkombination aus **1** und **2** können allein aus den genannten Wirkstoffen oder aus einem Gemisch der genannten Wirkstoffe mit physiologisch verträglichen Hilfsstoffen bestehen. Die erfindungsgemäßen Darreichungsformen können die Wirkstoffkombination aus **1** und **2** entweder gemeinsam in einer oder in zwei getrennten Darreichungsformen enthalten. Diese im Rahmen der vorliegenden Erfindung bevorzugt einsetzbaren Darreichungsformen werden im nachfolgenden Teil der Beschreibung detailliert beschrieben.

### Inhalationspulver enthaltend die erfindungsgemäßen Wirkstoffkombinationen aus 1 und 2:

Die erfindungsgemäßen Inhaltionspulver können **1** und **2** entweder allein oder im Gemisch mit geeigneten physiologisch unbedenkliche Hilfsstoffen enthalten.

Sind die Wirkstoffe **1** und **2** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1** und **2,** vorzugsweise mit einer mittleren Teilchengröße von 0,5 bis 10µm, besonders bevorzugt von 1 bis 6µm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt. Die erfindungsgemäßen Inhaltionspulver können entweder in Form einer einzigen Pulvermischung, die sowohl **1** als auch **2** enthält oder in Form von separaten Inhalationspulvern, die lediglich **1** und **2** enthalten bereitgestellt und appliziert werden.

Die erfindungsgemäßen Inhalationspulver können mittels aus dem Stand der Technik bekannten Inhalatoren appliziert werden.

Erfindungsgemäße Inhalationspulver, die neben **1** und **2** ferner einen physiologisch unbedenklichen Hilfsstoff enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Vorzugsweise werden die erfindungsgemäßen Inhalationspulver, die neben **1** und **2** physiologisch unbedenkliche Hilfsstoff enthalten, allerdings in Kapseln abgefüllt (zu sogenannten Inhaletten), die in Inhalatoren wie beispielsweise in der WO 94/28958 beschrieben, zur Anwendung gelangen.

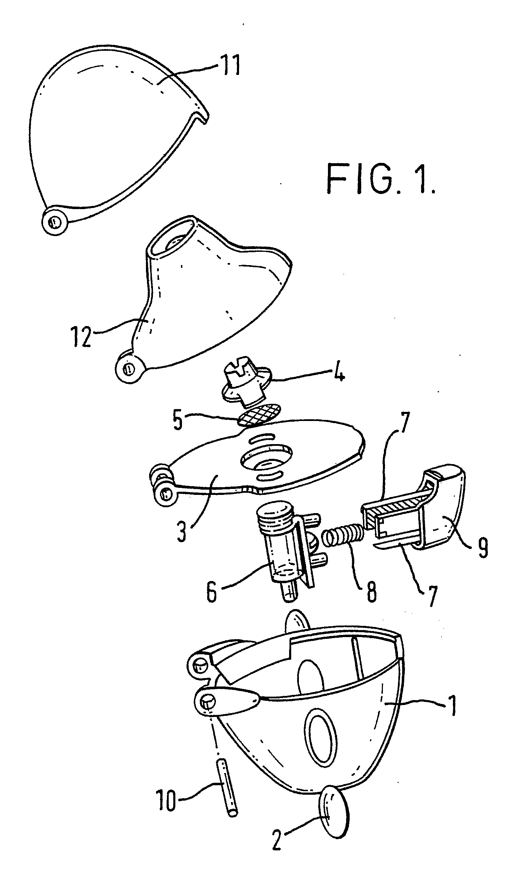

Ein zur Anwendung der erfindungsgemäßen Arzneimittelkombination in Inhaletten besonders bevorzugter Inhalator ist Figur 1 zu entnehmen.

Dieser Inhalator (Handihaler) für die Inhalation pulverförmiger Arzneimittel aus Kapseln ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12.

Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 30mg, bevorzugt von 3 bis 20mg, bevorzugt 5 bis 10 mg Inhalationspulver pro Kapsel an. Diese enthalten erfindungsgemäß entweder gemeinsam oder jeweils die bereits vorstehend für **1'** und **2'** genannten Dosierungen pro Einmalgabe.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### Formulierungsbeispiele

### A) Inhaltionspulver:

1)

| Bestandteile | µg pro Kapsel |
|---|---|
| Tiotropiumbromidmonohydrat | 10,8 |
| Salmeterol-salz (**2.1**) | 35 |
| Lactose | 4954,2 |
| Summe | 5000 |

2)

| Bestandteile | µg pro Kapsel |
|---|---|
| Tiotropiumbromidmonohydrat | 21,7 |
| Salmeterol-salz (**2.23**) | 75 |
| Lactose | 4903,3 |
| Summe | 5000 |

3)

| Bestandteile | µg pro Kapsel |
|---|---|
| Tiotropiumbromidmonohydrat | 22,5 |
| Salmeterol-salz (**2.1**) | 80,5 |
| Lactose | 4897 |
| Summe | 5000 |

4)

| Bestandteile | µg pro Kapsel |
|---|---|
| Tiotropiumbromid x H2O | 22,5 |
| Salmeterol-salz (**2.23**) | 95,5 |
| Lactose | 4828 |
| Summe | 5000 |

## Patentansprüche

1. Arzneimittel **gekennzeichnet durch** den Gehalt an einem oder mehreren Tiotropiumsalzen (**1**) in Kombination mit einem oder mehreren schwerlöslichen Salmeterolsalzen (**2**)**,** wobei die Salmeterolsalze **2** ausgewählt sind aus den Säureadditionssalzen des Salmeterols **2'** mit einer der nachstehend genannten Säuren: 2-Hydroxy-1-naphthoesäure, 3,5-Dichlorsalicylsäure, Furan-2-carbonsäure, 2,5-Dihydroxy-terephthalsäure, Zimtsäure, Triphenylessigsäure, 4-Phenylzimtsäure, Biphenyl-2-carbonsäure, 4-Trifluormethylzimtsäure, 9-Fluorenylidenessigsäure, 3-(2-Naphthyl)acrylsäure, 3-(1-Naphthyl)acrylsäure, 1-Naphthoesäure, 2,6-Dichlorbenzoesäure, 3,4-Dichlorbenzoesäure, 3,5-Dichlorbenzoesäure, 4-Brombenzoesäure, 4-Trifluormethylbenzoesäure, 4-Isopropylbenzoesäure, 4-tert.-Butylbenzoesäure, 3-(3-lndolyl)acrylsäure, 2,4-Dichlorzimtsäure, 2,6-Dichlorzimtsäure, 2,5-Dimethoxy-zimtsäure, 2-Trifluormethylzimtsäure, 3-Trifluormethylzimtsäure, 3-Chlorzimtsäure, 3,4-Dichlorzimtsäure, 4-Bromzimtsäure, 4-Chlorzimtsäure, 4-Methoxyzimtsäure, 4-Fluorzimtsäure, 4-Isopropylzimtsäure, 4-tert.-Butylzimtsäure, 2.6-Difluorzimtsäure, 2,4-Difluorzimtsäure, 3,4-Difluorzimtsäure, 2,4,5-Trifluorzimtsäure, 3,4,5-Trifluorzimtsäure, 3-Methoxysalicylsäure, 4-Methoxysalicylsäure, 5-Methoxysalicylsäure, 4-Methylsalicylsäure, 5-Aminosalicylsäure, 3-Chlorsalicylsäure, 5-Sulfosalicylsäure, 5-Acetylsalicylsäure, 3,5-Diiodsalicylsäure, Isochinolin-1-carbonsäure, 9-Fluorencarbonsäure, 9-Fluorenon-1 -carbonsäure, 3,5-Diisopropyl-salicylsäure oder Diflunisal; und in Wasser eine Löslichkeit von 0,1 mg/ml oder weniger aufweisen.

2. Arzneimittel nach Anspruch 1, **dadurch gekennzeichnet, daß** die Wirkstoffe **1** und **2** entweder gemeinsam in einer einzigen Darreichungsform oder in zwei getrennten Darreichungsformen enthalten sind.

3. Arzneimittel nach einem der Ansprüche 1 oder 2, **dadurch gekennzeichnet, daß 1** in Form des Chlorids, Bromids, lodids, Methansulfonats, para-Toluolsulfonats oder Methylsulfats, bevorzugt in Form des Bromids enthalten ist.

4. Arzneimittel nach Anspruch 1, **dadurch gekennzeichnet, daß 2** ausgewählt ist aus der Gruppe bestehend aus den folgenden Salzen:
4-Hydroxy-α¹-[[[6-(4-phenylbutoxy)-hexyl]-amino]-methyl]-1,3-benzoldimethanol-4-phenylcinnamat-Salz;
4-Hydroxy-α¹-[[[6-(4-phenylbutoxy)-hexyl]-amino]-methyl]-1,3-benzoldimethanol-4-trifluoromethyl-cinnamat-Salz;
4-Hydroxy-α¹-[[[6-(4-phenylbutoxy)-hexyl]-amino]-methyl]-1,3-benzoldimethanol-3,4-dichlor-cinnamat-Salz;
4-Hydroxy-α¹-[[[6-(4-phonylbutoxy)-hexyl]-amino]-methyl]-1,3-benzoldimethanol-2,4-dichlor-cinnamat-Salz;
4-Hydroxy-α¹-[[[6-(4-phenylbutoxy)-hexyl]-amino)-methyl]-1,3-benzoldimethanol-cinnamat-Salz;
4-Hydroxy-α¹-[[[6-(4-phenylbutoxy)-hexyl]-amino]-methyl]-1,3-benzoidimethanol-3-(2-naphthyl)acrylat-Salz;
4-Hydroxy-α¹-[[[6-(4-phenylbutoxy)-hexyl]-amino]-methyl]-1,3-benzoldimethanol-3-(1 -naphthyl)acrylat-Salz;
4-Hydroxy-α¹-[[[6-(4-phenylbutoxy)-hexyl]-amino]-methyl]-1,3-benzoldimethanol-2,6-dichlor-cinnamat-Salz;
4-Hydroxy-α¹-[[[6-(4-phenylbutoxy)-hexyl]-amino]-methyl]-13-benzοldimethanοl-2,5-dimethoxy-cιnnamat-Salz;
4-Hydroxy-α¹-[[[6-(4-phenylbutoxy)-hexyl]-amino]-methyl]-1,3-benzoldimethanol-2-trifluoromethyl-cinnamat-Salz;
4-Hydroxy-α¹-[[[6-(4-phenylbutoxy)-hexyl]-amino]-methyl]-1,3-benzoldimethanol-3-trifluoromethyl-cinnamat-Salz;
4-Hydroxy-α¹-[[[6-(4-phenylbutoxy)-hexyl]-amino]-methyl]-1,3-benzoldimethanol-3-chlor-cinnamat-Salz;
4-Hydroxy-α¹-[[[6-(4-phenylbutoxy)-hexyl)-amino]-methyl]-1,3-benzoldimethanol-4-brom-cinnamat-Salz;
4-Hydroxy-α¹-[[[6-(4-phenylbutoxy)-hexyl]-amino]-methyl]-1,3-benzoldimethanol-4-chlor-cinnamat-Salz;
4-Hydroxy-α¹-[[[6-(4-phenylbutoxy)-hexyl]-amino]-methyl]-1,3-benzoldimethanol-4-methoxy-cinnamat-Salz;
4-Hydroxy-α¹-[[[6-(4-phenylbutoxy)-hexyl]-amino]-methyl]-1,3-benzoldimethanol-4-fluor-cinnamat-Salz;
4-Hydroxy-α¹-[[[6-(4-phenylbutoxy)-hexyl]-amino]-methyl]-1,3-benzoldimethanol-4-isopropyl-cinnamat-Salz;
4-Hydroxy-α¹-[[[6-(4-phenylbutoxy)-hexyl]-amino]-methyl]-1,3-benzoldimethanol-4-tert-butyl-cinnamat-Salz;
4-Hydroxy-α¹-[[[6-(4-phenylbutoxy)-hexyl]-amino]-methyl]-1,3-benzoldimethanol-2,4-difluor-cinnamat-Salz;
4-Hydroxy-α¹-[[[6-(4-phenylbutoxy)-hexyl]-amino]-methyl]-1,3-benzoldimethanol-3.4-difluor-cinnamat-Salz;
4-Hydroxy-α¹-[[[6-(4-phenylbutoxy)-hexyl]-amino]-methyl]-1,3-benzoldimethanol-2,4,5-trifluor-cinnamat-Salz;
und
4-Hydroxy-α¹-[[[6-(4-phenylbutoxy)-hexyl]-amino]-methyl]-1,3-benzoldimethanol-3,4,5-trifluor-cinnamat-Salz.

5. Arzneimittel nach Anspruch 1, **dadurch gekennzeichnet, daß 2** ausgewählt ist aus der Gruppe bestehend aus den folgenden Salzen:
4-Hydroxy-α¹-[[[6-(4-phenylbutoxy)-hexyl]-amino]-methyl]-1,3-benzoidimethanol-5-(2,4-difluorphenyl)salicylat-Salz;
4-Hydroxy-α¹-[[[6-(4-phenylbutoxy)-hexyl]-amino]-methyl]-1,3-benzoldimethanol-3,5-diisopropyl-salicylat-Salz;
4-Hydroxy-α¹-[[[6-(4-phenylbutoxy)-hexyl]-amino]-methyl]-1,3-benzoldimethanol-4-chlor-salicylat-Salz;
4-Hydroxy-α¹-[[[6-(4-phenylbutoxy)-hexyl]-amino]-methyl]-1,3-benzoldimethanol-3,5-dichlor-salicylat-Salz;
4-Hydroxy-α¹-[[[6-(4-phenylbutoxy)-hexyl]-amino]-methyl]-1,3-benzoldimethanol-2,5-dihydroxyterephthalat-Salz (Base : Säure = 1:2);
4-Hydroxy-α¹-[[[6-(4-phenylbutoxy)-hexyl]-amino]-methyl]-1,3-benzoldimethanol-3-methoxysalicylat-Salz;
4-Hydroxy-α¹-[[[6-(4-phenylbutoxy)-hexyl]-amino]-methyl]-1,3-benzoldimethanol-4-methoxysalicylat-Salz;
4-Hydroxy-α¹-[[[6-(4-phenylbutoxy)-hexyl]-amino]-methyl]-1,3-benzoldimethanol-5-methoxysalicylat-Salz;
4-Hydroxy-α¹-[[[6-(4-phenylbutoxy)-hexyl]-amino]-methyl]-1,3-benzoldimethanol-4-methylsalicylat-Salz;
4-Hydroxy-α¹-[[[6-(4-phenylbutoxy)-hexyl]-amino]-methyl]-1,3-benzoldimethanol-5-aminosalicylat-Salz;
4-Hydroxy-α¹-[[[6-(4-phenylbutoxy)-hexyl]-amino]-methyl]-1,3-benzoldimethanol-3-chlorsalicylat-Salz;
4-Hydroxy-α¹-[[[6-(4-phenylbutoxy)-hexyl]-amino]-methyl]-1,3-benzoldimethanol-5-sulfo-salicylat-Salz;
4-Hydroxy-α¹-[[[6-(4-phenylbutoxy)-hexyl]amino]-methyl]-1,3-benzoldimethanol-5-acetylsalicylat-Salz;
und
4-Hydroxy-α¹-[[[6-(4-phenylbutoxy)-hexyl]-amino]-methyl]-1,3-benzoldimethanol-3,5-diiodsaticytat-Salz.

6. Arzneimittel nach einem der Ansprüche 1 bis 5, **dadurch gekennzeichnet, daß** die Gewichtsverhältnisse von Tiotropium **1'** zu Salmeterol **2'** ,in einem Bereich von 1:300 bis 30:1, bevorzugt von 1:230 bis 20:1 liegen.

7. Arzneimittel nach einem der Ansprüche 1 bis 6, **dadurch gekennzeichnet, daß** eine einmaliger Applikation einer Dosierung der Wirkstoffkombination **1'** und **2'** von 0,01 bis 1000µg, bevorzugt von 0,1 bis 200µg entspricht.

8. Arzneimittel nach einem der Ansprüche 1 bis 7, **dadurch gekennzeichnet, daß** es in Form eines für die Inhalation geeigneten Pulvers vorliegt.

9. Arzneimittel nach Anspruch 8, **dadurch gekennzeichnet, daß** es ein Inhalationspulver ist, welches **1** und **2** im Gemisch mit geeigneten physiologisch unbedenkliche Hilfsstoffen ausgewählt aus der Gruppe bestehend aus Monosaccharide, Disaccharide, Oligo- und Polysaccharide, Polyalkohole, Salze, oder Mischungen dieser Hilfsstoffe miteinandenthält.

10. Inhalationspulver nach Anspruch 9, **dadurch gekennzeichnet, daß** der Hilfsstoff eine maximale mittlere Teilchengröße von bis zu 250µm, bevorzugt zwischen 10 und 150 µm aufweist.

11. Kapseln **gekennzeichnet durch** einen Gehalt an Inhaltionspulver nach Anspruch 9 oder 10.

12. Arzneimittel nach Anspruch 8, **dadurch gekennzeichnet, daß** es ein Inhalationspulver ist, welches als Bestandteile lediglich die Wirkstoffe **1** und **2** enthält.

13. Verwendung einer Zusammensetzung nach einem der Ansprüche 1 bis 12 zur Herstellung eines Medikaments zur Behandlung von Atemwegserkrankungen.

14. Verwendung nach Anspruch 13 zur Herstellung eines Medikaments zur Behandlung von Asthma oder COPD.

## Claims

1. A pharmaceutical composition **characterized by** a content of one or more tiotropium salts (**1**) in combination with one or more sparingly soluble salmeterol salts (**2**), the salmeterol salts **2** being selected from the acid addition salts of salmeterol **2'** with one of the following acids: 2-hydroxy-1-naphthoic acid, 3,5-dichlorosalicylic acid, furan-2-carboxylic acid, 2,5-dihydroxyterephthalic acid, cinnamic acid, triphenylacetic acid, 4-phenylcinnamic acid, biphenyl-2-carboxylic acid, 4-trifluoromethylcinnamic acid, 9-fluorenylidene acetic acid, 3-(2-naphthyl)acrylic acid, 3-(1-naphthyl)acrylic acid, 1-naphthoic acid, 2,6-dichlorobenzoic acid, 3,4-dichlorobenzoic acid, 3,5-dichlorobenzoic acid, 4-bromobenzoic acid, 4-trifluoromethylbenzoic acid, 4-isopropylbenzoic acid, 4-tert-butylbenzoic acid, 3-(3-indolyl)acrylic acid, 2,4-dichlorocinnamic acid, 2,6-dichlorocinnamic acid, 2,5-dimethoxycinnamic acid, 2-trifluoromethylcinnamic acid, 3-trifluoromethylcinnamic acid, 3-chlorocinnamic acid, 3,4-dichlorocinnamic acid, 4-bromocinnamic acid, 4-chlorocinnamic acid, 4-methoxycinnamic acid, 4-fluorocinnamic acid, 4-isopropylcinnamic acid, 4-tert-butylcinnamic acid, 2,6-difluorocinnamic acid, 2,4-difluorocinnamic acid, 3,4-difluorocinnamic acid, 2,4,5-trifluorocinnamic acid, 3,4,5-trifluorocinnamic acid, 3-methoxysalicylic acid, 4-methoxysalicylic acid, 5-methoxysalicylic acid, 4-methylsalicylic acid, 5-aminosalicylic acid, 3-chlorosalicylic acid, 5-sulfosalicylic acid, 5-acetylsalicylic acid, 3,5-diiodosalicylic acid, isoquinoline-1-carboxylic acid, 9-fluorenecarboxylic acid, 9-fluorenone-1-carboxylic acid, 3,5-diisopropylsalicylic acid, or diflunisal; and having a solubility in water of 0.1 mg/mL or less.

2. The pharmaceutical composition according to Claim 1, **characterized in that** the active substances **1** and **2** are contained together in a single dosage form or in two separate dosage forms.

3. The pharmaceutical composition according to one of Claims 1 or 2, **characterized in that 1** is contained in the form of the chloride, bromide, iodide, methanesulfonate, para-toluenesulfonate, or methyl sulfate, preferably in the form of the bromide.

4. The pharmaceutical composition according to Claim 1, **characterized in that 2** is selected from the group comprising the following salts:
4-Hydroxy-α¹-[[[6-(4-phenylbutoxy)hexyl]amino]methyl]-1,3-benzenedimethanol-4-phenyl cinnamate salt;
4-Hydroxy-α¹-[[[6-(4-phenylbutoxy)hexyl]amino]methyl]-1,3-benzenedimethanol-4-trifluoromethyl cinnamate salt;
4-Hydroxy-α¹-[[[6-(4-phenylbutoxy)hexyl]amino]methyl]-1,3-benzenedimethanol-3,4-dichloro cinnamate salt;
4-Hydroxy-α¹-[[[6-(4-phenylbutoxy)hexyl]amino]methyl]-1,3-benzenedimethanol-2,4-dichloro cinnamate salt;
4-Hydroxy-α¹-[[[6-(4-phenylbutoxy)hexyl]amino]methyl)-1,3-benzenedimethanol cinnamate salt;
4-Hydroxy-α¹-[[[6-(4-phenylbutoxy)hexyl]amino]methyl]-1,3-benzenedimethanol-3-(2-naphthyl)acrylate salt;
4-Hydroxy-α¹-[[[6-(4-phenylbutoxy)hexyl]amino]methyl]-1,3-benzenedimethanol-3-(1-naphthyl)acrylate salt;
4-Hydroxy-α¹-[[[6-(4-phenylbutoxy)hexyl)amino]methyl]-1,3-benzenedimethanol-2,6-dichloro cinnamate salt;
4-Hydroxy-α¹-[[[6-(4-phenylbutoxy)hexyl]amino]methyl]-1,3-benzenedimethanol-2,5-dimethoxy cinnamate salt;
4-Hydroxy-α¹-[[[6-(4-phenylbutoxy)hexyl]amino]methyl]-1,3-benzenedimethanol-2-trifluoromethyl cinnamate salt;
4-Hydroxy-α¹-[[[6-(4-phenylbutoxy)hexyl]amino]methyl]-1,3-benzenedimethanol-3-trifluoromethyl cinnamate salt;
4-Hydroxy-α¹-[[[6-(4-phenylbutoxy)hexyl]aminomethyl]-1,3-benzenedimethanol-3-chloro cinnamate salt;
4-Hydroxy-α¹-[[[6-(4-phenylbutoxy)hexyl]amino]methyl]-1,3-benzenedimethanol-4-bromo cinnamate salt;
4-Hydroxy-α¹-[[[6-(4-phenylbutoxy)hexyl]amino]methyl]-1,3-benzenedimethanol-4-chloro cinnamate salt;
4-Hydroxy-α¹-[[[6-(4-phenylbutoxy)hexyl]amino]methyl]-1,3-benzenedimethanol-4-methoxy cinnamate salt;
4-Hydroxy-α¹-[[[6-(4-phenylbutoxy)hexyl]amino]methyl]-1,3-benzenedimethanol-4-fluoro cinnamate salt;
4-Hydroxy-α¹-[[[6-(4-phenylbutoxy)hexyl]amino]methyl]-1,3-benzenedimethanol-4-isopropyl cinnamate salt;
4-Hydroxy-α¹-[[[6-(4-phenylbutoxy)hexyl]amino]methyl]-1,3-benzenedimethanol-4-*tert*-butyl cinnamate salt;
4-Hydroxy-α¹-[[[6-(4-phenylbutoxy)hexyl]amino]methyl]-1,3-benzenedimethanol-2,4-difluoro cinnamate salt;
4-Hydroxy-α¹-[[[6-(4-phenylbutoxy)hexyl]amino]methyl]-1,3-benzenedimethanol-3,4-difluoro cinnamate salt;
4-Hydroxy-α¹-[[[6-(4-phenylbutoxy)hexyl]amino]methyl]-1,3-benzenedimethanol-2,4,5-trifluoro cinnamate salt;
and
4-Hydroxy-α¹-[[[6-(4-phenylbutoxy)hexyl]amino]methyl]-1,3-benzenedimethanol-3,4,5-trifluoro cinnamate salt.

5. The pharmaceutical composition according to Claim 1, **characterized in that 2** is selected from the group comprising the following salts:
4-Hydroxy-α¹-[[[6-(4-phenylbutoxy)hexyl]amino]methyl]-1,3-benzenedimethanol-5-(2,4-difluorophenyl) salicylate salt;
4-Hydroxy-α¹-[[[6-(4-phenylbutoxy)hexyl]amino]methyl]-1,3-benzenedimethanol-3, 5-diisopropyl salicylate salt;
4-Hydroxy-α¹-[[[6-(4-phenylbutoxy)hexyl]amino]methyl]-1,3-benzenedimethanol-4-chloro salicylate salt;
4-Hydroxy-α¹-[[[6-(4-phenylbutoxy)hexyl]amino]methyl]-1,3-benzenedimethanol-3,5-dichloro salicylate salt;
4-Hydroxy-α¹-[[[6-(4-phenylbutoxy)hexyl]amino]-methyi]-1,3-benzenedimethanol-2,5-dihydroxyterephthalate salt (base : acid = 1:2);
4-Hydroxy-α¹-[[[6-(4-phenylbutoxy)hexyl]amino]methyl]-1,3-benzenedimethanol-3-methoxy salicylate salt;
4-Hydroxy-α¹-[[[6-(4-phenylbutoxy)hexyl]amino]methyl]-1,3-benzenedimethanol-4-methoxy salicylate salt;
4-Hydroxy-α¹-[[[6-(4-phenylbutoxy)hexyl]amino]methyl]-1,3-benzenedimethanol-5-methoxy salicylate salt;
4-Hydroxy-α¹-[[[6-(4-phenylbutoxy)hexyl]amino]methyl]-1,3-benzenedimethanol-4-methyl salicylate salt;
4-Hydroxy-α¹-[([6-(4-phenylbutoxy)hexyl]amino]methyl]-1,3-benzenedimethanol-5-amino salicylate salt;
4-Hydroxy-α¹-[[[6-(4-phenylbutoxy)hexyl]amino]methyl]-1,3-benzenedimethanol-3-chloro salicylate salt;
4-Hydroxy-α¹-[[[6-(4-phenylbutoxy)hexyl]amino]methyl]-1,3-benzenedimethanol-5-sulfo salicylate salt;
4-Hydroxy-α¹-[[[6-(4-phenylbutoxy)hexyl]amino]methyl]-1,3-benzenedimethanol-5-acetyl salicylate salt;
and
4-Hydroxy-α¹-[[[6-(4-phenylbutoxy)hexyl]amino]methyl]-1,3-benzenedimethanol-3,5-diiodo salicylate salt.

6. The pharmaceutical composition according to one of Claims 1 to 5, **characterized in that** the weight ratios of tiotropium **1'** to salmeterol **2'** are in a range of 1:300 to 30:1, preferably 1:230 to 20:1.

7. The pharmaceutical composition according to one of Claims 1 to 6, **characterized in that** a single administration corresponds to a dosage of the active substance combination **1'** and **2'** of 0.01 to 1000 µg, preferably 0.1 to 200 µg.

8. The pharmaceutical composition according to one of Claims 1 to 7, **characterized in that** it is present in the form of a powder that is suitable for inhalation.

9. The pharmaceutical composition according to Claim 8, **characterized in that** it is an inhalable powder which contains **1** and **2** in a mixture with suitable physiologically acceptable excipients selected from the group comprising monosaccharides, disaccharides, oligo- and polysaccharides, polyalcohols, salts, or mixtures of these excipients.

10. The inhalable powder according to Claim 9, **characterized in that** the excipient has a maximum average particle size of up to 250 µm, preferably between 10 and 150 µm.

11. Capsules **characterized by** a content of inhalable powder according to Claim 9 or 10.

12. The pharmaceutical composition according to Claim 8, **characterized in that** it is an inhalable powder which contains only the active substances **1** and **2** as ingredients.

13. Use of a composition according to one of Claims 1 to 12 for preparing a medicament for treating respiratory diseases.

14. Use according to Claim 13 for preparing a medicament for treating asthma or COPD.

## Revendications

1. Médicament **caractérisé par** la teneur en un ou plusieurs sels de tiotropium **(1)** en combinaison avec un ou plusieurs sels de salmétérol **(2)** peu solubles, dans lequel les sels de salmétérol **2** sont choisis parmi les sels d'addition acide du salmétérol **2'** avec un des acides mentionnés ci-après : acide 2-hydroxy-1-naphtoïque, acide 3,5-dichlorosalicylique, acide furan-2-carboxylique, acide 2,5-dihydroxy-téréphtalique, acide cinnamique, acide triphénylacétique, acide 4-phénylcinnamique, acide biphényl-2-carboxyique, acide 4-trifluorométhylcinnamique, acide 9-fluorénylidène-acétique, acide 3-(2-naphtyl)acrylique, acide 3-(1-naphtyl)acrylique, acide 1-naphtoïque, acide 2,6-dichlorobenzoïque, acide 3,4-dichlorobenzoïque, acide 3,5-dichlorobenzoïque, acide 4-bromobenzoïque, acide 4-trifluorométhylbenzoïque, acide 4-isopropylbenzoïque, acide 4-tert-butylbenzoïque, acide 3-(3-indolyl)acrylique, acide 2,4-dichlorocinnamique, acide 2,6-dichlorocinnamique, acide 2,5-diméthoxy-cinnamique, acide 2-trifluorométhylcinnamique, acide 3-trifluorométhylcinnamique, acide 3-chlorocinnamique, acide 3,4-dichlorocinnamique, acide 4-bromocinnamique, acide 4-chlorocinnamique, acide 4-méthoxycinnamique, acide 4-fluorocinnamique, acide 4-isopropylcinnamique, acide 4-tert-butylcinnamique, acide 2,6-difluorocinnamique, acide 2,4-difluorocinnamique, acide 3,4-difluorocinnamique, acide 2,4,5-trifluoro-cinnamique, acide 3,4,5-trifluorocinnamique, acide 3-méthoxysalicylique, acide 4-méthoxysalicylique, acide 5-méthoxysalicylique, acide 4-méthylsalicylique, acide 5-aminosalicylique, acide 3-chlorosalicylique, acide 5-sulfosalicylique, acide 5-acétylsalicylique, acide 3,5-diiodosalicylique, acide isoquinolin-1-carboxylique, acide 9-fluorène-carboxylique, acide 9-fluorénon-1-carboxylique, acide 3,5-diisopropyl-salicylique ou diflunisal ; et présentent une solubilité dans l'eau de 0,1 mg/ml ou inférieure.

2. Médicament selon la revendication 1, **caractérisé en ce que** les substances actives **1** et **2** sont contenues conjointement dans une forme galénique unique ou dans deux formes galéniques séparées.

3. Médicament selon l'une des revendications 1 ou 2, **caractérisé en ce que** le composé **1** est contenu sous la forme de chlorure, bromure, iodure, méthanesulfonate, para-toluènesulfonate ou méthylsulfate, de préférence sous la forme de bromure.

4. Médicament selon la revendication 1, **caractérisé en ce que** le composé **2** est choisi dans le groupe consistant en les sels suivants :
sel 4-hydroxy-α¹-[[[6-(4-phénylbutoxy)-hexyl]-amino]-méthyl]-1,3-benzoldiméthanol-4-phénylcinnamate ;
sel 4-hydroxy-α¹-[[[6-(4-phénylbutoxy)-hexyl]-amino]-méthyl]-1,3-benzoldiméthanol-4-trifluorométhyl-cinnamate ;
sel 4-hydroxy-α¹-[[[6-(4-phénylbutoxy)-hexyl]-amino]-méthyl]-1,3-benzoldiméthanol-3,4-dichloro-cinnamate ;
sel 4-hydroxy-α¹-[[[6-(4-phénylbutoxy)-hexyl]-amino]-méthyl]-1,3-benzoldiméthanol-2,4-dichloro-cinnamate ;
sel 4-hydroxy-α¹-[[[6-(4-phénylbutoxy)-hexyl]-amino]-méthyl]-1,3-benzoldiméthanol-cinnamate ;
sel 4-hydroxy-α¹-[[[6-(4-phénylbutoxy)-hexyl]-amino]-méthyl]-1,3-benzoldiméthanol-3-(2-naphtyl)acrylate ;
sel 4-hydroxy-α¹-[[[6-(4-phénylbutoxy)-hexyl]-amino]-méthyl]-1,3-benzoldiméthanol-3-(1-naphtyl)acrylate ;
sel 4-hydroxy-α¹-[[[6-(4-phénylbutoxy)-hexyl]-amino]-méthyl]-1,3-benzoldiméthanol-2,6-dichloro-cinnamate ;
sel 4-hydroxy-α¹-[[[6-(4-phénylbutoxy)-hexyl]-amino]-méthyl]-1,3-benzoldiméthanol-2,5-diméthoxy-cinnamate ;
sel 4-hydroxy-α¹-[[[6-(4-phénylbutoxy)-hexyl]-amino]-méthyl]-1,3-benzoldiméthanol-2-trifluorométhyl-cinnamate ;
sel 4-hydroxy-α¹-[[[6-(4-phénylbutoxy)-hexyl]-amino]-méthyl]-1,3-benzoldiméthanol-3-trifluorométhyl-cinnamate ;
sel 4-hydroxy-α¹-[[[6-(4-phénylbutoxy)-hexyl]-amino]-méthyl]-1,3-benzoldiméthanol-3-chloro-cinnamate ;
sel 4-hydroxy-α¹-[[[6-(4-phénylbutoxy)-hexyl]-amino]-méthyl]-1,3-benzoldiméthanol-4-bromo-cinnamate ;
sel 4-hydroxy-α¹-[[[6-(4-phénylbutoxy)-hexyl]-amino]-méthyl]-1,3-benzoldiméthanol-4-chloro-cinnamate ;
sel 4-hydroxy-α¹-[[[6-(4-phénylbutoxy)-hexyl]-amino]-méthyl]-1,3-benzoldiméthanol-4-méthoxy-cinnamate ;
sel 4-hydroxy-α¹-[[[6-(4-phénylbutoxy)-hexyl]-amino]-méthyl]-1,3-benzoldiméthanol-4-fluoro-cinnamate ;
sel 4-hydroxy-α¹-[[[6-(4-phénylbutoxy)-hexyl]-amino]-méthyl]-1,3-benzoldiméthanol-4-isopropyl-cinnamate ;
sel 4-hydroxy-α¹-[[[6-(4-phénylbutoxy)-hexyl]-amino]-méthyl]-1,3-benzoldiméthanol-4-tert-butyl-cinnamate ;
sel 4-hydroxy-α¹-[[[6-(4-phénylbutoxy)-hexyl]-amino]-méthyl]-1,3-benzoldiméthanol-2,4-difluoro-cinnamate ;
sel 4-hydroxy-α¹-[[[6-(4-phénylbutoxy)-hexyl]-amino]-méthyl]-1,3-benzoldiméthanol-3,4-difluoro-cinnamate ;
sel 4-hydroxy-α¹-[[[6-(4-phénylbutoxy)-hexyl]-amino]-méthyl]-1,3-benzoldiméthanol-2,4,5-trifluoro-cinnamate ;
et
sel 4-hydroxy-α¹-[[[6-(4-phénylbutoxy)-hexyl]-amino]-méthyl]-1,3-benzoldiméthanol-3,4,5-trifluoro-cinnamate.

5. Médicament selon la revendication 1, **caractérisé en ce que** le composé **2** est choisi dans le groupe consistant en les sels suivants :
sel 4-hydroxy-α¹-[[[6-(4-phénylbutoxy)-hexyl]-amino]-méthyl]-1,3-benzoldiméthanol-5-(2,4-difluorophényl)salicylate ;
sel 4-hydroxy-α¹-[[[6-(4-phénylbutoxy)-hexyl]-amino]-méthyl]-1,3-benzoldiméthanol-3,5-diisopropyl-salicylate ;
sel 4-hydroxy-α¹-[[[6-(4-phénylbutoxy)-hexyl]-amino]-méthyl]-1,3-benzoldiméthanol-4-chloro-salicylate ;
sel 4-hydroxy-α¹-[[[6-(4-phénylbutoxy)-hexyl]-amino]-méthyl]-1,3-benzoldiméthanol-3,5-dichloro-salicylate ;
sel 4-hydroxy-α¹-[[[6-(4-phénylbutoxy)-hexyl]-amino]-méthyl]-1,3-benzoldiméthanol-2,5-dihydroxytéréphtalate (base:acide = 1:2) ;
sel 4-hydroxy-α¹-[[[6-(4-phénylbutoxy)-hexyl]-amino]-méthyl]-1,3-benzoldiméthanol-3-méthoxysalicylate ;
sel 4-hydroxy-α¹-[[[6-(4-phénylbutoxy)-hexyl]-amino]-méthyl]-1,3-benzoldiméthanol-4-méthoxysalicylate ;
sel 4-hydroxy-α¹-[[[6-(4-phénylbutoxy)-hexyl]-amino]-méthyl]-1,3-benzoldiméthanol-5-méthoxysalicylate ;
sel 4-hydroxy-α¹-[[[6-(4-phénylbutoxy)-hexyl]-amino]-méthyl]-1,3-benzoldiméthanol-4-méthylsalicylate ;
sel 4-hydroxy-α¹-[[[6-(4-phénylbutoxy)-hexyl]-amino]-méthyl]-1,3-benzoldiméthanol-5-amino-salicylate ;
sel 4-hydroxy-α¹-[[[6-(4-phénylbutoxy)-hexyl]-amino]-méthyl]-1,3-benzoldiméthanol-3-chloro-salicylate ;
sel 4-hydroxy-α¹-[[[6-(4-phénylbutoxy)-hexyl]-amino]-méthyl]-1,3-benzoldiméthanol-5-sulfosalicylate ;
sel 4-hydroxy-α¹-[[[6-(4-phénylbutoxy)-hexyl]-amino]-méthyl]-1,3-benzoldiméthanol-5-acétylsalicylate;
et
sel 4-hydroxy-α¹-[[[6-(4-phénylbutoxy)-hexyl]-amino]-méthyl]-1,3-benzoldiméthanol-3,5-diiodosalicylate.

6. Médicament selon l'une des revendications 1 à 5, **caractérisé en ce que** les rapports en poids du tiotropium **1'** au salmétérol **2'** se situent dans une plage de 1:300 à 30:1, de préférence de 1:230 à 20:1.

7. Médicament selon l'une des revendications 1 à 6, **caractérisé en ce qu'**une application unique d'un dosage de la combinaison de substance active **1'** et **2'** correspond à de 0,01 à 1000 µg, de préférence de 0,1 à 200 µg.

8. Médicament selon l'une des revendications 1 à 7, **caractérisé en ce qu'**il se présente sous la forme d'une poudre appropriée pour l'inhalation.

9. Médicament selon la revendication 8, **caractérisé en ce qu'**il est une poudre à inhaler qui maintient en mélange les composés **1** et **2** dans le mélange avec des adjuvants appropriés physiologiquement inoffensifs choisis dans le groupe consistant en monosaccharides, disaccharides, oligo- et polysaccharides, polyalcools, sels, ou des mélanges de ces adjuvants.

10. Poudre à inhaler selon la revendication 9, **caractérisé en ce que** l'adjuvant présente une taille de particules moyennes maximales allant jusqu'à 250 µm, de préférence entre 10 et 150 µm.

11. Capsules **caractérisées par** une teneur en poudre à inhaler selon la revendication 9 ou 10.

12. Médicament selon la revendication 8, **caractérisé en ce qu'**il est une poudre à inhaler qui contient comme composants, uniquement les substances actives **1** et **2.**

13. Utilisation d'une composition selon l'une des revendications 1 à 12 pour la fabrication d'un médicament pour le traitement de maladies des voies respiratoires.

14. Utilisation selon la revendication 13 pour la fabrication d'un médicament pour le traitement de l'asthme ou de la BPCO.
